(19) Europäisches Patentamt
European Patent Office
Office européen des brevets

(11) EP 1 736 156 A1

(12) **EUROPEAN PATENT APPLICATION**
published in accordance with Art. 158(3) EPC

(43) Date of publication:
**27.12.2006 Bulletin 2006/52**

(21) Application number: **05734263.6**

(22) Date of filing: **14.04.2005**

(51) Int Cl.:
***A61K 31/439*** *(2006.01)* ***A61K 9/14*** *(2006.01)*
***A61K 9/16*** *(2006.01)* ***A61K 9/20*** *(2006.01)*
***A61K 9/28*** *(2006.01)* ***A61K 9/48*** *(2006.01)*
***A61K 47/02*** *(2006.01)* ***A61K 47/04*** *(2006.01)*
***A61K 47/10*** *(2006.01)* ***A61K 47/12*** *(2006.01)*
***A61K 47/38*** *(2006.01)* ***A61P 1/00*** *(2006.01)*
*C07D 451/04* *(2006.01)*

(86) International application number:
**PCT/JP2005/007564**

(87) International publication number:
**WO 2005/099698 (27.10.2005 Gazette 2005/43)**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **15.04.2004 JP 2004120599**

(71) Applicant: **Eisai R&D Management Co., Ltd.**
**Tokyo 112-8088 (JP)**

(72) Inventors:
- **NAKATA, Hitoshi**
**c/o Kawashima Industrial Park**
**Kakamigahara-shi, Gifu (JP)**
- **ISHIDA, Atsunori**
**c/o Kawashima Industrial Park**
**Kakamigahara-shi, Gifu (JP)**

(74) Representative: **HOFFMANN EITLE**
**Patent- und Rechtsanwälte**
**Arabellastrasse 4**
**81925 München (DE)**

(54) **STABILIZED 4-AMINO-5-CHLORO-N-¬(1R,3r,5S)-8-METHYL-8- AZABICYCLO¬3.2.1|OCT-3-YL|-2-¬1-METHYLBUT-2-YNYLOXY|-BENZAMIDE CONTAINING COMPOSITION**

(57) The present invention provides stable pharmaceutical compositions containing 4-amino-5-chloro-N-[(1R,3r,5S)-8-methyl-8-azabicyclo[3.2.1]oct-3-yl]-2-[1-methylbut-2-ynyloxy]benzamide. Specifically, it provides pharmaceutical compositions containing 1) 4-amino-5-chloro-N-[(1R,3r,5S)-8-methyl-8-azabicyclo[3.2.1]oct-3-yl]-2-[1-methylbut-2-ynyloxy]benzamide, a pharmacologically acceptable salt thereof or a hydrate of them; and 2) a basic additive.

EP 1 736 156 A1

## Description

### Technical Field

The present invention relates to a stabilized pharmaceutical composition containing 4-amino-5-chloro-N-[(1R, 3r,5S)-8-methyl-8-azabicyclo[3.2.1]oct-3-yl]-2-[1-methylbut-2-ynyloxy]benzamide, a pharmacologically acceptable salt thereof or a hydrate of them in combination with a basic additive.

### Background Art

The proportion of patients with unidentified complaints such as abdominal full-consciousness, heartburn, nausea and vomiting among those with gastrointestinal diseases has reached 60% or above. Most of such unidentified complaints are caused by gastrointestinal dysfunction. Japanese Patent No. 2721631 discloses 4-amino-5-chloro-N-[(1R,3r,5S)-8-methyl-8-azabicyclo[3.2.1]-oct-3-yl]-2-[1-methylbut-2-ynyloxy]benzamide (also known as endo-4-amino-5-chloro-N-[8-methyl-8-azabicyclo[3.2.1]oct-3-yl]-2-{(3-pentyn-2-yl)oxy}benzamide), and its isomer 4-amino-5-chloro-N-[(1R,3r,5S)-8-methyl-8-azabicyclo[3.2.1]-oct-3-yl]-2-[(1S)-1-methylbut-2-ynyloxy]benzamide. This compound is disclosed as an aminobenzoic acid derivative having a strong gastrointestinal prokinetic action due to its 5-HT4 agonistic action and having a strong inhibitory action on vomiting and nausea due to its 5-HT3 receptor antagonistic action. The compound is expected to have efficacy to anorexia, protracted nausea and vomiting, upon which other 5-HT3 receptor antagonists are not so effective.

Pharmaceutical compositions containing the active ingredient and regular pharmaceutical additives, however, cannot significantly maintain their stability over a long time. The compound is insufficient in stability in an aqueous solution at elevated temperatures, and most of pharmaceutical additives contain water or moisture. Accordingly, there is no other way but to wrap solid pharmaceutical compositions containing the present compound and general pharmaceutical additives in moisture-proof packaging or pack them with drying agent such as silica gel in order to ensure their stability during long-term storage.

### Disclosure of Invention

Demands have been made on pharmaceutical compositions that contain 4-amino-5-chloro-N-[(1R,3r,5S)-8-methyl-8-azabicyclo[3.2.1]oct-3-yl]-2-[1-methylbut-2-ynyloxy]-benzamide and are stable even during long-term storage. This compound has a strong gastrointestinal prokinetic action due to 5-HT4 agonistic action and has a strong inhibitory action on vomiting and nausea due to 5-HT3 receptor antagonistic action. More strong demands have been made on those containing, its isomer, 4-amino-5-chloro-N-[(1R,3r,5S)-8-methyl-8-azabicyclo[3.2.1]oct-3-yl]-2-[(1S)-1-methylbut-2-ynyloxy]benzamide. Such solid pharmaceutical compositions must be more stable without the need of desiccants and moisture-proof packaging.

Under these circumstances, the present inventors made intensive investigations on pharmaceutical compositions containing 4-amino-5-chloro-N-[(1R,3r,5S)-8-methyl-8-azabicyclo[3.2.1]oct-3-yl]-2-[1-methylbut-2-ynyloxy]-benzamide, especially on those containing 4-amino-5-chloro-N-[(IR,3r,5S)-8-methyl-8-azabicyclo[3.2.1]oct-3-yl]-2-[(1S)-1-methylbut-2-ynyloxy]benzamide, in order to find stable solid pharmaceutical preparations that ensure the quality of the drug over a long time. As a result, they have found that the expected object can be achieved by the following configuration. The present invention has been achieved based on these findings.

The present invention provides a pharmaceutical composition comprising 1) 4-amino-5-chloro-N-[(1R,3r,5S)-8-methyl-8-azabicyclo[3.2.1]oct-3-yl]-2-[1-methylbut-2-ynyloxy]benzamide represented by the following formula (I), a pharmacologically acceptable salt thereof or a hydrate of them; and 2) a basic additive.

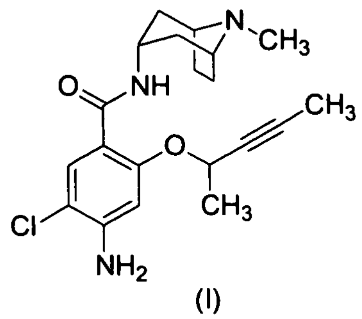

(I)

Detailed Description of the Invention

The 4-amino-5-chloro-N-[(1R,3r,5S)-8-methyl-8-azabicyclo[3.2.1]oct-3-yl]-2-[1-methylbut-2-ynyloxy]-benzamide relating to the present invention includes, as its isomers, 4-amino-5-chloro-N-[(1R,3r,5S)-8-methyl-8-azabicyclo [3.2.1]oct-3-yl]-2-[(1S)-1-methylbut-2-ynyloxy]-benzamide and 4-amino-5-chloro-N-[(1R,3r,5S)-8-methyl-8-azabicyclo [3.2.1]oct-3-yl]-2-[(1R)-1-methylbut-2-ynyloxy]-benzamide, of which 4-amino-5-chloro-N-[(1R,3r,5S)-8-methyl-8-azabicyclo[3.2.1]oct-3-yl]-2-[(1S)-1-methylbut-2-ynyloxy]benzamide represented by the following formula (II) is preferred.

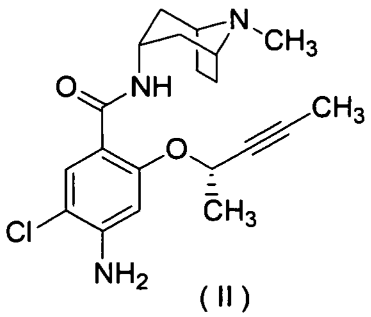

( II )

The 4-amino-5-chloro-N-[(1R,3r,5S)-8-methyl-8-azabicyclo[3.2.1]oct-3-yl]-2-[1-methylbut-2-ynyloxy]-benzamide and 4-amino-5-chloro-N-[(1R,3r,5S)-8-methyl-8-azabicyclo[3.2.1]oct-3-yl]-2-[(1S)-1-methylbut-2-ynyloxy]-benzamide can each be a pharmacologically acceptable salt thereof or a hydrate of them. Such pharmacologically acceptable salts include, but are not limited to, salts of inorganic acids such as hydrochlorides, sulfates, phosphates or hydrobromides; salts of organic acids such as acetates, methanesulfonates, tartrates, maleates, benzenesulfonates or toluenesulfonates; and salts of amino acids such as glutamates or aspartates.

The basic additive for use in the present invention is not specifically limited, as long as an aqueous solution or suspension thereof has a pH of 7 or more. Of such basic additives, those having a solubility in water of 0.1% or less are preferred. Preferred examples thereof are 1) hydroxide or oxide of magnesium or calcium, and 2) calcium or magnesium salt of a suitable inorganic or organic weak acid including phosphoric acid, a carboxylic acid or citric acid, such as magnesium hydroxide, magnesium oxide, calcium hydroxide, calcium oxide, calcium carbonate, calcium phosphate, calcium sulfate, magnesium carbonate, magnesium phosphate, magnesium sulfate, calcium citrate or aminoalkyl methacrylate copolymer E. The basic additive is more preferably magnesium hydroxide, magnesium oxide, calcium hydroxide, calcium carbonate, calcium phosphate or aminoalkyl methacrylate copolymer E, of which magnesium hydroxide or magnesium oxide is more preferred.

The amount of the basic additive for use in the present invention is not specifically limited and is generally 1 to 300 parts by weight, preferably 5 to 120 parts by weight, and more preferably 20 to 80 parts by weight, to 1 part by weight of 4-amino-5-chloro-N-[(1R,3r,5S)-8-methyl-8-azabicyclo[3.2.1]oct-3-yl]-2-[1-methylbut-2-ynyloxy]benzamide, a pharmacologically acceptable salt thereof or a hydrate of them. The amount is generally 1% by weight to 50% by weight, preferably 3% by weight to 30% by weight, and more preferably 5% by weight to 15% by weight, based on the weight of the pharmaceutical composition containing 4-amino-5-chloro-N-[(1R,3r,5S)-8-methyl-8-azabicyclo[3.2.1]oct-3-yl]-2-[1-methylbut-2-ynyloxy]benzamide, a pharmacologically acceptable salt thereof or a hydrate of them. The amount of the basic additive is not limited to the above-specified ranges.

The pharmaceutical compositions according to the present invention may further comprise general or regular

pharmaceutical additives such as fillers, disintegrators, binders and/or lubricants.

The fillers include, but are not limited to, sugar alcohols such as mannitol, inositol, maltitol, xylitol or erythritol; saccharides such as trehalose, corn starch, lactose, partially pregelatinized starch, sucrose, α-cyclodextrin, β-cyclodextrin or γ-cyclodextrin; crystalline cellulose; and anhydrous dibasic calcium phosphate. Among them, sugar alcohols such as mannitol, inositol, maltitol, xylitol or erythritol are preferred, of which mannitol is more preferred. Mannitol is typically preferred to be incorporated into pharmaceutical compositions containing 4-amino-5-chloro-N-[(1R,3r,5S)-8-methyl-8-azabicyclo [3.2.1]-oct-3-yl]-2-[1-methylbut-2-ynyloxy]benzamide. This is because the compound is unstable in an aqueous solution at elevated temperatures, but mannitol has a very low moisture content. The amount of the fillers is not specifically limited and is preferably 40% by weight to 99.9% by weight, more preferably 60% by weight to 99% by weight, and particularly preferably 65% by weight to 95% by weight based on the weight of the pharmaceutical composition.

The disintegrators include, but are not limited to, low substituted hydroxypropylcellulose, croscarmellose sodium, carboxymethylcellulose, carboxymethylcellulose calcium, crospovidone, hydroxypropyl starch and sodium carboxymethyl starch, of which low substituted hydroxypropylcellulose is preferred. The amount of the disintegrators is not specifically limited, and is preferably 3% by weight to 30% by weight, more preferably 5% by weight to 20% by weight, and particularly preferably 5% by weight to 15% by weight based on the weight of the pharmaceutical composition.

The binders include, but are not limited to, hydroxypropyl methylcellulose, methylcellulose, hydroxypropyl cellulose, poly(vinyl alcohol)s and polyvinylpyrrolidones, of which hydroxypropyl methylcellulose is preferred. The amount of the binders is not specifically limited and is preferably 1% by weight to 5% by weight, more preferably 1.5% by weight to 4% by weight, and particularly preferably 1.5% by weight to 3% by weight based on the weight of the pharmaceutical composition.

The lubricants include, but are not limited to, magnesium stearate, calcium stearate, stearic acid, sodium stearyl fumarate, hydrogenated castor oil and talc. Among them, magnesium stearate and sodium stearyl fumarate are preferred, of which sodium stearyl fumarate is more preferred. When the basic additive is magnesium oxide or magnesium hydroxide, and magnesium stearate is used as a lubricant in a pharmaceutical composition in the form of, for example, a tablet, the tablet may often have a prolonged disintegration time and a reduced rate of dissolution due to gelation. In contrast, when sodium stearyl fumarate is used as the lubricant, gelation does not occur, the pharmaceutical composition such as a tablet has improved wettability to a solution and shows a shortened disintegration time and an increased rate of dissolution. The amount of the lubricants is not specifically limited and is preferably 0.1% by weight to 2% by weight, more preferably 0.2% by weight to 1.8% by weight, and particularly preferably 0.2 to 1.5% by weight, based on the weight of the pharmaceutical composition.

The pharmaceutical compositions according to the present invention may further comprise refrigerants, sweeteners and other substances. Examples of the refrigerants are 1-menthol and mentha water. Examples of the sweeteners are aspartame, stevia extract, licorice root, saccharin, saccharin sodium, dipotassium glycyrrhizinate and glycine.

The dosage forms of the pharmaceutical compositions according to the present invention include, but are not limited to, solid pharmaceutical preparations such as tablets, capsules, powders, granules, fine granules, dry syrups, troches, inhalants, suppositories, ointments or tapes; and liquid pharmaceutical preparations such as nasal drops, ear drops, cataplasms or lotions. Pharmaceutical preparations of these dosage forms can be prepared according to conventional procedures. Among these pharmaceutical preparations, solid pharmaceutical preparations such as tablets, capsules, powders, granules, fine granules, dry syrups, troches, inhalants, suppositories, ointments or tapes are preferred, of which tablets, capsules, powders, granules and fine granules are more preferred.

The pharmaceutical compositions according to the present invention can be prepared according to a conventional procedure. For example, granules, subtle granules or powders can be prepared by incorporating 4-amino-5-chloro-N-[(1R,3r,5S)-8-methyl-8-azabicyclo[3.2.1]-oct-3-yl]-2-[1-methylbut-2-ynyloxy]benzamide, a pharmacologically acceptable salt thereof or a hydrate of them with a basic additive such as magnesium hydroxide or magnesium oxide as a stabilizer; further adding an excipient, a disintegrator, and/or a binder; subjecting the mixture to dry granulation typically using a roller compactor, or wet granulation such as tumbling granulation or extrusion granulation; drying the granulated substance; and sizing the dried substance. It is also acceptable that the granulated substance is further combined with a disintegrator and/or a lubricant, and the mixture is charged into capsules to yield capsules or is compressed into tablets. Tablets can also be prepared by incorporating 4-amino-5-chloro-N-[(1R,3r,5S)-8-methyl-8-azabicyclo[3.2.1]oct-3-yl]-2-[1-methylbut-2-ynyloxy]-benzamide, a pharmacologically acceptable salt thereof, or a hydrate of them with a basic additive such as magnesium hydroxide or magnesium oxide as a stabilizer; further incorporating the mixture with an excipient, a disintegrator, a binder, and/or a lubricant; and subjecting the mixture to direct compression (direct tablet making). Of course, the production procedure is not limited to these procedures.

The tablets, capsules, powders, granules, and subtle granules according to the present invention may be coated with a coating film formed according to a conventional procedure, for the purpose typically of protection from light, masking of bitter taste, or ensuring sustainability. Of these preparations, tablets, granules and fine granules are more suitable for coating. The coating film may comprise various additives in addition to a coating agent. The coating can be carried out, for example, by incorporating a coating agent with additives such as plasticizers, dispersing agents or

colorants; dissolving or dispersing the mixture in a medium such as water, ethanol, isopropyl alcohol, or a mixture of these media; and subjecting the solution or dispersion to coating in a fluidized bed coater, a coating pan, or a centrifugal fluidized bed granulator.

The coating agent includes, but is not limited to, hydroxypropyl methylcellulose, methylcellulose, hydroxypropyl cellulose, poly(vinyl alcohol)s, polyvinylpyrrolidones, ethyl cellulose, methacrylic acid/acrylic acid copolymers (e.g., trade name: Eudragit (Rohm, Germany), methacrylic acid copolymer of Type A, methacrylic acid copolymer of Type B and methacrylic acid copolymer of Type C). The plasticizer includes, but is not limited to, polyethylene glycol, triethyl citrate and glycerol fatty acid esters. The dispersing agent includes, but is not limited to, magnesium stearate, calcium stearate and talc. The coating composition may comprise a colorant. Examples of the colorant are yellow ferric oxide, yellow oxide of iron, Food Yellow No. 4, Food Yellow No. 5, Food Yellow No. 4 aluminum lake and sesquioxide, Food Red No. 2, Food Red No. 3 and Food Red No. 102, sodium copper chlorophyllin, potassium copper chlorophyllin and titanium oxide.

Each of these can be used alone or in combination in the present invention.

The present invention further provides a method for producing a stabilized composition containing 4-amino-5-chloro-N-[(1R,3r,5S)-8-methyl-8-azabicyclo[3.2.1]oct-3-yl]-2-[1-methylbut-2-ynyloxy]benzamide, comprising the step of incorporating 1) 4-amino-5-chloro-N-[(1R,3r,5S)-8-methyl-8-azabicyclo[3.2.1]oct-3-yl]-2-[1-methylbut-2-ynyloxy]-benzamide represented by Formula (I), a pharmacologically acceptable salt thereof or a hydrate of them with 2) a basic additive. The basic additive preferably stands in contact with 4-amino-5-chloro-N-[(1R,3r,5S)-8-methyl-8-azabicyclo[3.2.1]oct-3-yl]-2-[1-methylbut-2-ynyloxy]-benzamide, a pharmacologically acceptable salt thereof or a hydrate of them. These two components may be homogenously mixed or may be in different layers in contact with each other. They are more preferably homogenously mixed.

The present invention further provides a method for stabilizing 4-amino-5-chloro-N-[(1R,3r,5S)-8-methyl-8-azabicyclo[3.2.1]oct-3-yl]-2-[1-methylbut-2-ynyloxy]-benzamide, comprising the step of incorporating 1) 4-amino-5-chloro-N-[(1R,3r,5S)-8-methyl-8-azabicyclo[3.2.1]oct-3-yl]-2-[1-methylbut-2-ynyloxy]benzamide represented by the formula (I), a pharmacologically acceptable salt thereof or a hydrate of them with 2) a basic additive.

The 4-amino-5-chloro-N-[(1R,3r,5S)-8-methyl-8-azabicyclo[3.2.1]oct-3-yl]-2-[1-methylbut-2-ynyloxy]-benzamide is preferably 4-amino-5-chloro-N-[(1R,3r,5S)-8-methyl-8-azabicyclo[3.2.1]oct-3-yl]-2-[(1S)-1-methylbut-2-ynyloxy] benzamide represented by the formula (II).

The present invention has been achieved based on findings that pharmaceutical compositions containing 4-amino-5-chloro-N-[(1R,3r,5S)-8-methyl-8-azabicyclo[3.2.1]-oct-3-yl]-2-[1-methylbut-2-ynyloxy]benzamide as an active ingredient can be very satisfactorily stabilized by incorporating the active ingredient with basic additives; and that, of such basic additives, those having a low solubility in water are more effective, of which magnesium hydroxide, magnesium oxide, calcium hydroxide, calcium carbonate, calcium phosphate and aminoalkyl methacrylate copolymer E can surprisingly effectively stabilize the active ingredient.

The pharmaceutical compositions according to the present invention can be prepared, for example, by the following procedure.

4-Amino-5-chloro-N-[(1R,3r,5S)-8-methyl-8-azabicyclo-[3.2.1]oct-3-yl]-2-[1-methylbut-2-ynyloxy]benzamide (7 g), low substituted hydroxypropylcellulose (182 g) and magnesium oxide (54.6 g) are mixed using a high shear type mixture and thereby yield a triturated powder mixture. The triturated powder mixture (237.8 g), mannitol granules for direct compression prepared by spraying (1506.9 g), and magnesium stearate (32 g) are admixed using a bin type mixer, the mixture is subjected to tablet making (compression) with a tablet making machine, and thereby yields tablets each having a weight of 65 mg and each containing 0.25 mg of 4-amino-5-chloro-N-[(1R,3r,5S)-8-methyl-8-azabicyclo[3.2.1]oct-3-yl]-2-[1-methylbut-2-ynyloxy]benzamide. According to this procedure, excellently stale pharmaceutical compositions containing 4-amino-5-chloro-N-[(1R,3r,5S)-8-methyl-8-azabicyclo[3.2.1]-oct-3-yl]-2-[1-methylbut-2-ynyloxy]benzamide can be prepared.

Where necessary, coated tablets can be prepared by coating 400 g of the tablets with an aqueous solution of hydroxypropyl methylcellulose having a solid content of 10% and containing homogenously dispersed titanium oxide, magnesium stearate and polyethylene glycol.

The present invention enables excellent stability of pharmaceutical compositions containing 4-amino-5-chloro-N-[(1R,3r,5S)-8-methyl-8-azabicyclo[3.2.1]oct-3-yl]-2-[1-methylbut-2-ynyloxy]benzamide. Examples of advantages thereof are illustrated below.

Experimental Examples

(1) Stabilization action of basic additive incorporated into powdery pharmaceutical preparation (pharmaceutical composition) containing 4-amino-5-chloro-N-[(1R,3r,5S)-8-methyl-8-azabicyclo[3.2.1]oct-3-yl]-2-[(1S)-1-methylbut-2-ynyloxy] benzamide

A) Compatibility test of 4-amino-5-chloro-N-[(1R,3r,5S)-8-methyl-8-azabicyclo[3.2.1]oct-3-yl]-2-[(1S)-1-methylbut-2-ynyloxy]benzamide and pharmaceutical additives

Compatibility tests between 4-amino-5-chloro-N-[(1R,3r,5S)-8-methyl-8-azabicyclo[3.2.1]oct-3-yl]-2-[(1S)-1-methylbut-2-ynyloxy]benzamide and various pharmaceutical additives were conducted in the preparation of pharmaceutical preparations (pharmaceutical compositions) containing 4-amino-5-chloro-N-[(1R,3r,5S)-8-methyl-8-azabicyclo [3.2.1]oct-3-yl]-2-[(1S)-1-methylbut-2-ynyloxy]-benzamide. Specifically, 4-amino-5-chloro-N-[(1R,3r,5S)-8-methyl-8-azabicyclo[3.2.1]oct-3-yl]-2-[(1S)-1-methylbut-2-ynyloxy]benzamide was admixed with various pharmaceutical additives in weight proportions in Table 2, the resulting compositions were weighed in transparent glass bottles, and were stored at 60°C, relative humidity of 75% for one week while keeping the lid open. Various fillers, disintegrators, binders, lubricants, and others were used as the pharmaceutical additives. As a control, 4-amino-5-chloro-N-[(1R,3r,5S)-8-methyl-8-azabicyclo[3.2.1]oct-3-yl]-2-[(1S)-1-methylbut-2-ynyloxy]benzamide alone was weighed in a transparent glass bottle and was stored under the above-mentioned conditions. The samples before and after storage were evaluated using high-performance liquid chromatography (HPLC). The stabilities of the samples were evaluated based on the total amount of decomposition products (%), the amount (%) of main decomposition product at a retention time (RRT) of 1.37, and the residual ratio of content (%) of the active ingredient after storage. The amounts of individual decomposition products, the amount of main decomposition product at a retention time (RRT) of 1.37, and the content of the active ingredient were determined by the external standard method using a standard solution of the active ingredient.

Total amount of decomposition products (%) = Total sum of the amounts of individual decomposition products

Residual ratio of content (%) = [(Content after storage)/(Content before storage)] x 100

(HPLC condition)

The respective samples before and after storage were mixed with an extraction medium, stirred for sixty minutes, and centrifuged at 3000 rpm for fifteen minutes. The resulting supernatants were assayed by liquid chromatography to thereby determine the amounts of decomposition products and the content of the active ingredient.
Extraction medium: 30% by volume aqueous solution of acetonitrile
Detector: Ultraviolet absorptiometer
Mobile phase A: Mixture of water/acetonitrile/(sodium perchlorate monohydrate)/(perchloric acid) (900:100:7:1, V/V/W/V)
Mobile phase B: Mixture of acetonitrile/water/(sodium perchlorate monohydrate)/(perchloric acid) (900:100:7:1, V/V/W/V)
Column: Stainless steel tube having an inner diameter of 4.6 mm and a length of 15 cm, charged with 3-μm octadecylsilylated silica gel for liquid chromatography Column temperature: Constant temperature around 40°C Flow rate: 1 ml/minute

Table 1

| Time (min) | Ratio of the mobile phase B (%) | Concentration of acetonitrile (%) |
|---|---|---|
| 0 | 24 | 29 |
| 12 | 24 | 29 |
| 22 | 100 | 86 |

(continued)

| Time (min) | Ratio of the mobile phase B (%) | Concentration of acetonitrile (%) |
|---|---|---|
| 35 | 100 | 86 |

The results are shown in Table 2.

Table 2

| | Additive rate to the active ingredient | Pharmaceutical additive | Amount of main decomposition product at RRT=1.37 (%) | Total amount of decomposition products (%) | Residual ratio of content (%) |
|---|---|---|---|---|---|
| Filler | 100 | Mannitol | 0.76 | 0.82 | 99.0 |
| | 100 | Lactose | 0.31 | 0.91 | 100.0 |
| | 100 | Crystalline cellulose | 8.36 | 9.27 | 72.5 |
| | 100 | Com starch | 6.64 | 7.67 | 91.6 |
| Disintegrating agent | 100 | Partially pregelatinized starch | 6.76 | 7.61 | 92.9 |
| | 100 | Sodium carboxymethyl starch | 13.97 | 14.99 | 61.4 |
| | 100 | Croscarmellose sodium | 1.41 | 3.01 | 23.8 |
| | 100 | Carboxymethylcellulose | 0.60 | 1.63 | 17.9 |
| | 100 | Carboxymethylcellulose calcium | 4.29 | 5.35 | 81.8 |
| | 100 | Low substituted hydroxypropylcellulose | 2.69 | 2.69 | 77.2 |
| | 100 | Crospovidone | 2.16 | 14.69 | 70.4 |
| Binding agent | 50 | Hydroxypropyl methylcellulose | 0.79 | 1.26 | 99.3 |
| | 50 | Hydroxypropyl cellulose | 1.97 | 3.32 | 86.4 |
| | 50 | Polyvinylpyrrolidone | 2.48 | 11.80 | 79.3 |
| Lubricant | 15 | Magnesium stearate | 0.06 | 0.06 | 107.3 |
| | 15 | Sodium stearyl fumarate | 0.00 | 0.00 | 103.1 |
| (Control Example) | - | None (the active ingredient used alone) | 0.00 | 0.00 | 99.6 |

The powdery 4-amino-5-chloro-N-[(1R,3r,5S)-8-methyl-8-azabicyclo[3.2.1]oct-3-yl]-2-[(1S)-1-methylbut-2-ynyloxy]-benzamide is stable by itself, and there was observed no decomposition product (control). However, the samples comprising the active ingredient and regular pharmaceutical additives in Table 2 each showed an increased total amount of decomposition products, an increased amount of main decomposition product at a retention time (RRT) of 1.37, and a decreased residual ratio of content of the active ingredient. Of the excipients, the incorporation of mannitol or lactose caused less decomposition. Of the disintegrators, the incorporation of low substituted hydroxypropylcellulose, croscarmellose sodium or carboxymethylcellulose caused less decomposition. Of the binders, the incorporation of hydroxypropyl methylcellulose caused less decomposition products, and of the lubricants, the incorporation of magnesium stearate or sodium stearyl fumarate caused less decomposition products.

B) Evaluation of stabilities of powdery pharmaceutical preparations (pharmaceutical compositions) containing 4-amino-5-chloro-N-[(1R,3r,5S)-8-methyl-8-azabicyclo[3.2.1]-oct-3-yl]-2-[(1S)-1-methylbut-2-ynyloxy]benzamide, pharmaceutical additive, and basic additive

Powdery pharmaceutical preparations (pharmaceutical compositions) were prepared using basic additives obtained according to following Examples 1 to 5. The basic additives used were magnesium hydroxide (Example 1), magnesium oxide (Example 2), calcium carbonate (Example 3), sodium citrate (Example 4) and calcium phosphate (Example 5).

Each 250 mg of the granulated powdery pharmaceutical preparations (pharmaceutical compositions) were placed in polypropylene tubes and were stored at 60°C, relative humidity of 75% for one week while keeping the lid open. The samples before and after storage were evaluated by HPLC under the above-mentioned conditions. As comparative examples, a powdery pharmaceutical preparation incorporated with no stabilizer (Comparative Example 1), powdery pharmaceutical preparations incorporated with sodium carbonate and meglumine as basic additives, respectively (Comparative Examples 2 and 3), a powdery pharmaceutical preparation incorporated with a neutral additive (sodium chloride) (Comparative Example 4) and a powdery pharmaceutical preparation incorporated with an acidic additive (citric acid) (Comparative Example 5) were prepared and evaluated by the procedure of Examples 1 to 5. The stabilities of the samples were evaluated based on the total amount of decomposition products (%) and the amount of main decomposition product at a retention time (RRT) of 1.37 (%) after storage. The total amount of decomposition products and the amount of main decomposition product at a retention time (RRT) of 1.37 were determined according to the following expressions.

Total amount of decomposition products (%) = [(Total sum of peak areas of decomposition products after storage)/(Peak area of the active ingredient before storage)] x 100

Amount of main decomposition product at a retention time of 1.37 (%) = [(Peak area of main decomposition product at a retention time of 1.37 after storage)/(Peak area of the active ingredient before storage)] x 100

The formulations and the results of stability tests are shown in Tables 3 and 4, respectively.

Table 3

| Formula (mg) | | | | | | | | | | |
|---|---|---|---|---|---|---|---|---|---|---|
| Components | Example 1 | Example 2 | Example 3 | Example 4 | Example 5 | Comparative Example 1 | Comparative Example 2 | Comparative Example 3 | Comparative Example 4 | Comparative Example 5 |
| Drug | 0.2 | 0.2 | 0.2 | 0.2 | 0.2 | 0.2 | 0.2 | 0.2 | 0.2 | 0.2 |
| Mannitol | 177.8 | 177.8 | 177.8 | 177.8 | 177.8 | 177.8 | 177.8 | 177.8 | 177.8 | 177.8 |
| L-HPC | 20.0 | 20.0 | 20.0 | 20.0 | 20.0 | 20.0 | 20.0 | 20.0 | 20.0 | 20.0 |
| Magnesium stearate | 2.0 | 2.0 | 2.0 | 2.0 | 2.0 | 2.0 | 2.0 | 2.0 | 2.0 | 2.0 |
| Magnesium hydroxide | 50.0 | - | - | - | - | - | - | - | - | - |
| Magensium oxide | - | 50.0 | - | - | - | - | - | - | - | - |
| Sodium carbonate | - | - | - | - | - | - | 50.0 | - | - | - |
| Meglumine | - | - | - | - | - | - | - | 50.0 | - | - |
| Calcium carbonate | - | - | 50.0 | - | - | - | - | - | - | - |
| Sodium citrate | - | - | - | 50.0 | - | - | - | - | - | - |
| Calcium phosphate | - | - | - | - | 50.0 | - | - | - | - | - |
| Sodium chloride | - | - | - | - | - | - | - | - | 50.0 | - |
| Citric acid | - | - | - | - | - | - | - | - | - | 50.0 |
| Total weight (mg) | 250.0 | 250.0 | 250.0 | 250.0 | 250.0 | 200.0 | 250.0 | 250.0 | 250.0 | 250.0 |

Table 4

| | Basic additive | Amount of main decomposition product at RRT=1.37 (%) | Total amount of decomposition products (%) |
|---|---|---|---|
| Example 1 | Magnesium hydroxide | 1.21 | 3.41 |
| Example 2 | Magensium oxide | 1.90 | 4.15 |
| Example 3 | Calcium carbonate | 3.05 | 5.08 |
| Example 4 | Sodium citrate | 4.40 | 5.72 |
| Example 5 | Calcium phosphate | 4.48 | 6.73 |
| Comparative Example 1 | None | 5.06 | 7.68 |
| Comparative Example 2 | Sodium carbonate | 0.24 | 10.47 |
| Comparative Example 3 | Meglumine | 0.95 | 10.86 |
| Comparative Example 4 | Sodium chloride | 3.45 | 9.41 |
| Comparative Example 5 | Citric acid | 11.80 | 25.79 |

Of powdery pharmaceutical preparations (pharmaceutical compositions) containing 4-amino-5-chloro-N-[(1R,3r,5S)-8-methyl-8-azabicyclo[3.2.1]oct-3-yl]-2-[(1S)-1-methylbut-2-ynyloxy]benzamide, a pharmaceutical additive and a basic additive, those using magnesium hydroxide (Example 1), magnesium oxide (Example 2), calcium carbonate (Example 3), sodium citrate (Example 4) and calcium phosphate (Example 5) showed a decreased amount of main decomposition product at a retention time (RRT) of 1.37 and a decreased total amount of decomposition products, as compared with the powdery pharmaceutical preparation containing no basic additive (Comparative Example 1). Among them, those using magnesium hydroxide or magnesium oxide were significantly effectively stabilized.

In contrast, the powdery pharmaceutical preparations using basic additives such as sodium carbonate (Comparative Example 2) and meglumine (Comparative Example 3) were more unstable as compared with the powdery pharmaceutical preparation containing no basic additive (Comparative Example 1), even though these basic additives have a pH substantially equal to that of the basic additives used in Examples 1 to 5. Specifically, these powdery pharmaceutical preparations were unstabilized, because they showed an increased total amount of decomposition products, although they showed a decreased amount of main decomposition product at a retention time (RRT) of 1.37. The powdery pharmaceutical preparations incorporated with sodium chloride as a neutral additive (Comparative Example 4) and citric acid as an acidic additive (Comparative Example 5), respectively, showed further accelerated decomposition.

These results clearly show that pharmaceutical compositions containing 4-amino-5-chloro-N-[(1R,3r,5S)-8-methyl-8-azabicyclo[3.2.1]oct-3-yl]-2-[(1S)-1-methylbut-2-ynyloxy]benzamide are stabilized by incorporating basic additives, of which basic additives having a low solubility in water of, for example, 0.1% or less, such as magnesium hydroxide, magnesium oxide, calcium carbonate, sodium citrate or calcium phosphate, are more effective.

(2) Stabilization effect and dosage effect of basic additives on tablets (pharmaceutical compositions) containing 4-amino-5-chloro-N-[(1R,3r,5S)-8-methyl-8-azabicyclo[3.2.1]oct-3-yl]-2-[(1S)-1-methylbut-2-ynyloxy]-benzamide

Tablets incorporated with basic additives and prepared according to following Examples 6 to 15 were placed in plastic bottles and were stored at 60°C, relative humidity of 75% for one week. The samples after storage were evaluated by HPLC under the above-mentioned conditions.

The tablets were prepared by blending of powders and direct compression using, as basic additives, 10% of magnesium hydroxide (Example 6), 10% of aminoalkyl methacrylate copolymer E (Example 7), 10% of calcium hydroxide (Example 8), and 3%, 6.5%, and 10% of magnesium oxide (Examples 9 to 11), respectively, based on the weight of the pharmaceutical composition. According to Example 12, tablets were prepared by adding 10% of magnesium oxide, subjecting the mixture to wet granulation, and then to tablet making (compression), and the resulting tablets were evaluated. According to Example 13, film-coated tablets were prepared by spraying a coating composition containing magnesium oxide to uncoated tablets prepared according to Comparative Example 7. According to Examples 14 and 15, film-coated tablets containing 0.30% and 2.38% of the active ingredient, respectively, were prepared by incorporating 9.5% of magnesium oxide, subjecting the mixture to wet granulation, carrying out tablet making of the granules, and coating the tablets. As comparative examples, tablets were prepared by blending powders and direct compression without incorporating a basic additive (Comparative Examples 6 and 7), and the tablets were evaluated in the same manner as above. The stabilities of these samples were evaluated based on the amount (%) of main decomposition product at a retention time (RRT) of 1.37 after storage. The amount of main decomposition product at a retention time

(RRT) of 1.37 was determined by the external standard method using a standard solution of the active ingredient.

The formulations and the results of the stability tests are shown in Tables 5 and 6, respectively.

Table 5

| Formula (mg) | | | | | | | | | | | | |
|---|---|---|---|---|---|---|---|---|---|---|---|---|
| Components | Example 6 | Example 7 | Example 8 | Example 9 | Example 10 | Example 11 | Example 12 | Example 13 | Example 14 | Example 15 | Comparative Example 6 | Comparative Example 7 |
| Drug | 0.25 | 0.25 | 0.25 | 0.25 | 0.25 | 0.25 | 0.25 | 0.25 | 0.25 | 2.00 | 0.25 | 0.25 |
| Mannitol | 50.55 | 50.55 | 50.55 | 55.10 | 53.80 | 50.55 | 52.10 | 57.05 | 56.95 | 55.20 | 60.30 | 57.05 |
| L-HPC | 6.5 | 6.5 | 6.5 | 6.5 | 6.5 | 6.5 | 3.25 | 6.50 | 12.0 | 12.0 | 3.25 | 6.5 |
| Magnesium stearate | 1.2 | 1.2 | 1.2 | 1.2 | 1.2 | 1.2 | 1.0 | 1.2 | - | - | 1.2 | 1.2 |
| Sodium stearyl fumarate | - | - | - | - | - | - | - | - | 0.8 | 0.8 | - | - |
| TC-5RW | - | - | - | - | - | - | 1.9 | - | 2.0 | 2.0 | - | - |
| Magnesium hydroxide | 6.5 | - | - | - | - | - | - | - | - | - | - | - |
| Aminoalkyl methacrylate copolymer E | - | 6.5 | - | - | - | - | - | - | - | - | - | - |
| Calcium hydroxide | - | - | 6.5 | - | - | - | - | - | - | - | - | - |
| Magnesium oxide | - | - | - | 1.95 | 3.25 | 6.5 | 6.5 | - | 8.0 | 8.0 | - | - |
| TC-5RW | - | - | - | - | - | - | - | 2.8 | 2.5 | 2.5 | - | - |
| Magnesium stearate | - | - | - | - | - | - | - | 1.2 | 1.0 | 1.0 | - | - |
| PEG 8000. | - | - | - | - | - | - | - | 0.5 | 0.44 | 0.44 | - | - |
| Yellow oxide of iron | - | - | - | - | - | - | - | - | 0.06 | 0.06 | - | - |
| Magnesium oxide | - | - | - | - | - | - | - | 0.5 | - | - | - | - |

(continued)

| Formula (mg) | | | | | | | | | | | | |
|---|---|---|---|---|---|---|---|---|---|---|---|---|
| Components | Example 6 | Example 7 | Example 8 | Example 9 | Example 10 | Example 11 | Example 12 | Example 13 | Example 14 | Example 15 | Comparative Example 6 | Comparative Example 7 |
| Total weight (mg) | 65 | 65 | 65 | 65 | 65 | 65 | 65 | 70 | 84 | 84 | 65 | 65 |

Table 6

| | | Titer of the active ingredient | Additive rate | Basic additive | Additive rate | Amount of main decomposition product at RRT=1.37 (%) |
|---|---|---|---|---|---|---|
| Direct tableting | Example 6 | 0.25mg | 0.38% | Magnesium hydroxide | 10% | 0.50 |
| | Example 7 | 0.25mg | 0.38% | Aminoalkyl methacrylate copolymer E | 10% | 0.27 |
| | Example 8 | 0.25mg | 0.38% | Calcium hydroxide | 10% | 0.04 |
| | Example 9 | 0.25mg | 0.38% | Magnesium oxide | 3% | 0.41 |
| | Example 10 | 0.25mg | 0.38% | Magnesium oxide | 6.5% | 0.26 |
| | Example 11 | 0.25mg | 0.38% | Magnesium oxide | 10% | 0.17 |
| Direct tableting and then coating | Example 12 | 0.25mg | 0.36% | Magnesium oxide (added only to the coating part) | 0.7% | 0.52 |
| Wet granulation and then tableting | Example 13 | 0.25mg | 0.38% | Magnesium oxide | 10% | 0.13 |
| Wet granulation, tableting and then coating | Example 14 | 0.25mg | 0.30% | Magnesium oxide | 9.5% | 0.11 |
| | Example 15 | 2mg | 2.38% | Magnesium oxide | 9.5% | 0.06 |
| Direct tableting | Comparative Example 6 | 0.25mg | 0.38% | None | | 0.61 |
| | Comparative Example 7 | 0.25mg | 0.38% | None | | 0.84 |

Of the tablets containing 4-amino-5-chloro-N-[(1R,3r,5S)-8-methyl-8-azabicyclo[3.2.1]oct-3-yl]-2-[(1S)-1-methylbut-2-ynyloxy]benzamide, a pharmaceutical additive, and a basic additive, those using, as the basic additive, 10% of magnesium hydroxide (Example 6), 10% of aminoalkyl methacrylate copolymer E (Example 7), 10% of calcium hydroxide (Example 8), and 10% of magnesium oxide (Example 11), respectively, showed a reduced amount of main decomposition product at a retention time (RRT) of 1.37, as compared with the powdery pharmaceutical preparation using no basic additive (Comparative Example 7). Table 5 shows the amount of main decomposition product at a retention time (RRT) of 1.37.

The amount of main decomposition product at a retention time (RRT) of 1.37 decreases with an increasing amount of magnesium oxide of 0.7%, 3%, 6.5% and 10% (Examples 9 to 11 and 13) based on the weight of the pharmaceutical composition.

These results reveal that pharmaceutical compositions containing 4-amino-5-chloro-N-[(1R,3r,5S)-8-methyl-8-azabicyclo[3.2.1]oct-3-yl]-2-[(1S)-1-methylbut-2-ynyloxy]-benzamide are stabilized by incorporating basic additives; that of such basic additives, those having a low solubility in water of, for example, 0.1% or less, such as magnesium hydroxide, aminoalkyl methacrylate copolymer E or calcium hydroxide, are more effective; and that the preparations are more stabilized with an increasing amount of the basic additives. In other words, the stabilization effect of the basic additives increases with an increasing amount thereof.

Examples

The present invention will be illustrated in further detail with reference to Examples below, which by no means limit the scope of the present invention.

Examples 1 to 5

Mannitol (890 g), low substituted hydroxypropylcellulose (100 g) and magnesium stearate (10 g) were mixed using a high shear type mixture, and the mixture was further combined with a solution of 1 g of 4-amino-5-chloro-N-[(1R,3r,5S)-8-methyl-8-azabicyclo[3.2.1]oct-3-yl]-2-[(1S)-1-methylbut-2-ynyloxy]benzamide in 100 g of ethanol, followed by gentle granulation. Next, 800 mg of the above-prepared granulated substance and 200 mg of a basic additive were subjected to gentle granulation in a mortar while adding 200 μL of purified water and thereby yielded fine granules containing 4-amino-5-chloro-N-[(1R,3r,5S)-8-methyl-8-azabicyclo[3.2.1]oct-3-yl]-2-[(1S)-1-methylbut-2-ynyloxy]benzamide.

The fine granules have a weight ratio of 4-amino-5-chloro-N-[(1R,3r,5S)-8-methyl-8-azabicyclo[3.2.1]oct-3-yl]-2-[(1S)-1-methylbut-2-ynyloxy]benzamide to the basic additive of 1:250.

The basic additives used herein were magnesium hydroxide (Example 1), magnesium oxide (Example 2), calcium carbonate (Example 3), sodium citrate (Example 4) and calcium phosphate (Example 5).

Example 6

A pre-mixture was prepared by mixing 7 g of 4-amino-5-chloro-N-[(1R,3r,5S)-8-methyl-8-azabicyclo[3.2.1]oct-3-yl]-2-[(1S)-1-methylbut-2-ynyloxy]benzamide, 182 g of low substituted hydroxypropylcellulose and 182 g of magnesium hydroxide using a high shear type mixture. The pre-mixture (367.3 g), mannitol granules for direct compression prepared by spraying (Parteck M200, produced by Merck) (1402.1 g) and magnesium stearate (32.4 g) were admixed using a bin type mixer, the mixture was subjected to tablet making (compression) with a 6-mm punch in a tablet making machine, and thereby yielded tablets each having a weight of 65 mg and containing 0.25 mg of 4-amino-5-chloro-N-[(1R,3r,5S)-8-methyl-8-azabicyclo[3.2.1]oct-3-yl]-2-[(1S)-1-methylbut-2-ynyloxy]benzamide.

Example 7

A pre-mixture was prepared by mixing 7 g of the active ingredient, 182 g of low substituted hydroxypropylcellulose and 185 g of aminoalkyl methacrylate copolymer E (Eudragit, manufactured by Rohm) using a high shear type mixture. The pre-mixture (364.5 g), mannitol granules for direct compression prepared by spraying (Parteck M200, produced by Merck) (1391.4 g) and magnesium stearate (32.2 g) were admixed using a bin type mixer, the mixture was subjected to tablet making (compression) with a 6-mm punch in a tablet making machine, and thereby yielded tablets each having a weight of 65 mg and containing 0.25 mg of 4-amino-5-chloro-N-[(1R,3r,5S)-8-methyl-8-azabicyclo[3.2.1]oct-3-yl]-2-[(1S)-1-methylbut-2-ynyloxy]benzamide.

Example 8

A pre-mixture was prepared by mixing 7 g of the active ingredient, 182 g of low substituted hydroxypropylcellulose and 182 g of potassium hydroxide using a high shear type mixture. The pre-mixture (360.9 g), mannitol granules for direct compression prepared by spraying (Parteck M200, produced by Merck) (1377.7 g) and magnesium stearate (31.9 g) were admixed using a bin type mixer, the mixture was subjected to tablet making (compression) with a 6-mm punch in a tablet making machine, and thereby yielded tablets each having a weight of 65 mg and containing 0.25 mg of 4-amino-5-chloro-N-[(1R,3r,5S)-8-methyl-8-azabicyclo-[3.2.1]oct-3-yl]-2-[(1S)-1-methylbut-2-ynyloxy]benzamide.

Example 9

A pre-mixture was prepared by mixing 7 g of the active ingredient, 182 g of low substituted hydroxypropylcellulose and 54.6 g of magnesium oxide using a high shear type mixture. The pre-mixture (237.8 g), mannitol granules for direct compression prepared by spraying (Parteck M200, produced by Merck) (1506.9 g) and magnesium stearate (32 g) were admixed using a bin type mixer, the mixture was subjected to tablet making (compression) with a 6-mm punch in a tablet making machine, and thereby yielded tablets each having a weight of 65 mg and containing 0.25 mg of 4-amino-5-chloro-N-[(1R,3r,5S)-8-methyl-8-azabicyclo-[3.2.1]oct-3-yl]-2-[(1S)-1-methylbut-2-ynyloxy]benzamide.

Example 10

A pre-mixture was prepared by mixing 7 g of the active ingredient, 182 g of low substituted hydroxypropylcellulose and 118.3 g of magnesium oxide using a high shear type mixture. The pre-mixture (301.9 g), mannitol granules for direct compression prepared by spraying (Parteck M200, produced by Merck) (1453.9 g) and magnesium stearate (32.2 g) were admixed using a bin type mixer, the mixture was subjected to tablet making (compression) with a 6-mm punch in a tablet making machine, and thereby yielded tablets each having a weight of 65 mg and containing 0.25 mg of 4-amino-5-chloro-N-[(1R,3r,5S)-8-methyl-8-azabicyclo-[3.2.1]oct-3-yl]-2-[(1S)-1-methylbut-2-ynyloxy]benzamide.

Example 11

A pre-mixture was prepared by mixing 7 g of the active ingredient, 182 g of low substituted hydroxypropylcellulose and 182 g of magnesium oxide using a high shear type mixture. The pre-mixture (366 g), mannitol granules for direct compression prepared by spraying (Parteck M200, produced by Merck) (1397.2 g) and magnesium stearate (32.3 g) were admixed using a bin type mixer, the mixture was subjected to tablet making (compression) with a 6-mm punch in a tablet making machine, and thereby yielded tablets each having a weight of 65 mg and containing 0.25 mg of 4-amino-5-chloro-N-[(1R,3r,5S)-8-methyl-8-azabicyclo-[3.2.1]oct-3-yl]-2-[(1S)-1-methylbut-2-ynyloxy]benzamide.

Example 12

Granules were prepared by mixing 7.5 g of the active ingredient, 1563 g of mannitol, 97.5 g of low substituted hydroxypropylcellulose, 195 g of magnesium oxide and 57 g of hydroxypropyl methylcellulose (TC-5RW, produced by Shin-Etsu Chemical Co., Ltd.) using a high shear type mixture, adding 440 g of purified water to the mixture, and carrying out granulation. The granulated substance was dried and subjected to sizing. The sized substance (1699.9 g) and magnesium stearate (26.6 g) were admixed using a bin type mixer, the mixture was subjected to tablet making (compression) with a 6-mm punch in a tablet making machine, and thereby yielded tablets each having a weight of 65 mg and each containing 0.25 mg of 4-amino-5-chloro-N-[(1R,3r,5S)-8-methyl-8-azabicyclo[3.2.1]oct-3-yl]-2-[(1S)-1-methylbut-2-ynyloxy]benzamide.

Example 13

Film-coated tablets each having a weight of 70 mg and each containing 0.25 mg of 4-amino-5-chloro-N-[(IR, 3r,5S)-8-methyl-8-azabicyclo[3.2.1]oct-3-yl]-2-[(1S)-1-methylbut-2-ynyloxy]benzamide were prepared by spraying a coating composition having the following composition to 250 g of the uncoated tablets prepared according to Comparative Example 7 using a pan-coating machine.

(Composition of coating composition)

| | |
|---|---|
| Hydroxypropylmethylcellulose (TC-5RW, produced by Shin-Etsu Chemical Co., Ltd.) | 5.6% |
| Magnesium stearate | 2.4% |
| Polyethylene glycol 8000 | 1.0% |
| Magnesium oxide | 1.0% |
| Purified water | 90.0% |

Example 14

Granules were prepared by mixing 6.25 g of the active ingredient, 1423.75 g of mannitol, 200 g of low substituted hydroxypropylcellulose, 200 g of magnesium oxide and 50 g of hydroxypropyl methylcellulose (TC-5RW, produced by Shin-Etsu Chemical Co., Ltd.) using a high shear type mixture, adding 560 g of purified water to the mixture, and carrying out granulation. The granulated substance was dried and subjected to sizing. The sized substance (1713.9 g), low substituted hydroxypropylcellulose (91.2 g) and sodium stearyl fumarate (18.2 g) were admixed using a bin type mixer, the mixture was subjected to tablet making (compression) with a 6-mm punch in a tablet making machine, and thereby yielded tablets each having a weight of 80 mg. Further, film-coated tablets each having a weight of 84 mg and each containing 0.25 mg of 4-amino-5-chloro-N-[(1R,3r,5S)-8-methyl-8-azabicyclo[3.2.1]oct-3-yl]-2-[(1S)-1-methylbut-2-ynyloxy]benzamide were prepared by spraying a coating composition having the following composition to 250 g of the

uncoated tablets using a pan-coating machine.

(Composition of coating composition)

| | |
|---|---|
| Hydroxypropylmethylcellulose (TC-5RW, produced by Shin-Etsu Chemical Co., Ltd.) | 5.0% |
| Magnesium stearate | 2.0% |
| Polyethylene glycol 8000 | 0.88% |
| Yellow oxide of iron | 0.12% |
| Purified water | 92.0% |

Example 15

Granules were prepared by mixing 50 g of the active ingredient, 1380 g of mannitol, 200 g of low substituted hydroxypropylcellulose, 200 g of magnesium oxide and 50 g of hydroxypropyl methylcellulose using a high shear type mixture, adding 560 g of purified water to the mixture, and carrying out granulation. The granulated substance was dried and subjected to sizing. The sized substance (1760.4 g) and magnesium stearate (26.6) were admixed using a bin type mixer, the mixture was subjected to tablet making (compression) with a 6-mm punch in a tablet making machine, and thereby yielded tablets each having a weight of 65 mg and each containing 0.25 mg of 4-amino-5-chloro-N-[(1R,3r,5S)-8-methyl-8-azabicyclo[3.2.1]oct-3-yl]-2-[(1S)-1-methylbut-2-ynyloxy]benzamide.

(Composition of coating composition)

| | |
|---|---|
| Hydroxypropyl methylcellulose (TC-5RW, produced by Shin-Etsu Chemical Co., Ltd.) | 5.0% |
| Magnesium stearate | 2.0% |
| Polyethylene glycol 8000 | 0.88% |
| Yellow oxide of iron | 0.12% |
| Purified water | 92.0% |

## Claims

**1.** A pharmaceutical composition comprising 1) 4-amino-5-chloro-N-[(1R,3r,5S)-8-methyl-8-azabicyclo[3.2.1]-oct-3-yl]-2-[1-methylbut-2-ynyloxy]benzamide represented by following formula (I), a pharmacologically acceptable salt thereof or a hydrate of them; and 2) a basic additive.

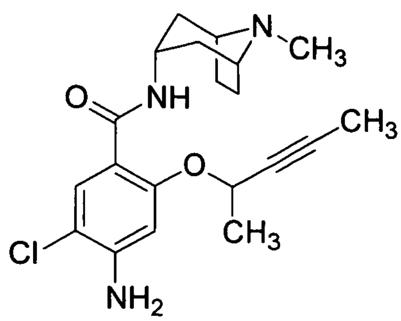

(I)

**2.** The pharmaceutical composition according to claim 1, wherein the 4-amino-5-chloro-N-[(1R,3r,5S)-8-methyl-8-azabicyclo[3.2.1]oct-3-yl]-2-[1-methylbut-2-ynyloxy]-benzamide is 4-amino-5-chloro-N-[(1R,3r,5S)-8-methyl-8-azabicyclo[3.2.1]oct-3-yl]-2-[(1S)-1-methylbut-2-ynyloxy]-benzamide represented by the following formula (II):

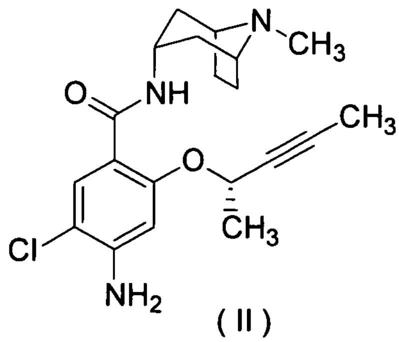

( II )

3. The pharmaceutical composition according to one of claims 1 and 2, further comprising at least one of a filler, a disintegrator, a binder and a lubricant.

4. The pharmaceutical composition according to any one of claims 1 to 3, wherein the basic additive is a basic additive having a solubility in water of 0.1% or less.

5. The pharmaceutical composition according to any one of claims 1 to 4, wherein the basic additive is one of magnesium hydroxide, magnesium oxide, calcium hydroxide, calcium carbonate, calcium phosphate and aminoalkyl methacrylate copolymer E.

6. The pharmaceutical composition according to claim 5, wherein the basic additive is one of magnesium hydroxide and magnesium oxide.

7. The pharmaceutical composition according to any one of claims 1 to 6, wherein the amount of the basic additive is 1 to 300 parts by weight to 1 part by weight of 4-amino-5-chloro-N-[(1R,3r,5S)-8-methyl-8-azabicyclo-[3.2.1]oct-3-yl]-2-[1-methylbut-2-ynyloxy]benzamide, a pharmacologically acceptable salt thereof or a hydrate of them.

8. The pharmaceutical composition according to any one of claims 3 to 7, wherein the filler is a sugar alcohol.

9. The pharmaceutical composition according to any one of claims 4 to 8, wherein the amount of the filler is 40% by weight to 99.9% by weight based on the weight of the pharmaceutical composition.

10. The pharmaceutical composition of one of claims 8 and 9, wherein the sugar alcohol is mannitol.

11. The pharmaceutical composition according to any one of claims 3 to 10, wherein the disintegrator is a low substituted hydroxypropylcellulose.

12. The pharmaceutical composition according to any one of claims 3 to 11, wherein the binder is hydroxypropyl methylcellulose.

13. The pharmaceutical composition according to any one of claims 3 to 12, wherein the lubricant is sodium stearyl fumarate or magnesium stearate.

14. The pharmaceutical composition according to claim 13, wherein the lubricant is sodium stearyl fumarate.

15. The pharmaceutical composition according to any one of claims 1 to 14, wherein the pharmaceutical composition is one of tablets, capsules, powder, granules and fine granules.

16. The pharmaceutical composition according to any one of claims 1 to 14, wherein the pharmaceutical composition is one of tablets, granules and fine granules, each coated with a coating film.

17. A method for producing a stabilized composition containing 4-amino-5-chloro-N-[(1R,3r,5S)-8-methyl-8-azabicyclo [3.2.1]oct-3-yl]-2-[1-methylbut-2-ynyloxy]-benzamide, comprising the step of incorporating 1) 4-amino-5-chloro-N-[(1R,3r,5S)-8-methyl-8-azabicyclo[3.2.1]oct-3-yl]-2-[1-methylbut-2-ynyloxy]benzamide represented by the formula (I), a pharmacologically acceptable salt thereof or a hydrate of them with 2) a basic additive.

**18.** A method for stabilizing 4-amino-5-chloro-N-[(1R,3r,5S)-8-methyl-8-azabicyclo[3.2.1]oct-3-yl]-2-[1-methylbut-2-ynyloxy]benzamide, comprising the step of incorporating 1) 4-amino-5-chloro-N-[(1R,3r,5S)-8-methyl-8-azabicyclo [3.2.1]oct-3-yl]-2-[1-methylbut-2-ynyloxy]benzamide represented by the formula (I), a pharmacologically acceptable salt thereof or a hydrate of them with 2) a basic additive.

**19.** The production method according to claim 17, wherein the 4-amino-5-chloro-N-[(1R,3r,5S)-8-methyl-8-azabicyclo [3.2.1]oct-3-yl]-2-[1-methylbut-2-ynyloxy]-benzamide is 4-amino-5-chloro-N-[(1R,3r,5S)-8-methyl-8-azabicyclo [3.2.1]oct-3-yl]-2-[(1S)-1-methylbut-2-ynyloxy]-benzamide represented by the formula (II).

**20.** The stabilization method according to claim 18, wherein the 4-amino-5-chloro-N-[(IR,3r,5S)-8-methyl-8-azabicyclo [3.2.1]oct-3-yl]-2-[1-methylbut-2-ynyloxy]-benzamide is 4-amino-5-chloro-N-[(1R,3r,5S)-8-methyl-8-azabicyclo [3.2.1]oct-3-yl]-2-[(1S)-1-methylbut-2-ynyloxy]-benzamide represented by the formula (II).

## INTERNATIONAL SEARCH REPORT

International application No.
PCT/JP2005/007564

A. CLASSIFICATION OF SUBJECT MATTER
Int.Cl7 A61K31/439, 9/14, 9/16, 9/20, 9/28, 9/48, 47/02, 47/04, 47/10, 47/12, 47/38, A61P1/00//C07D451/04

According to International Patent Classification (IPC) or to both national classification and IPC

B. FIELDS SEARCHED

Minimum documentation searched (classification system followed by classification symbols)
Int.Cl7 A61K31/439, 9/14, 9/16, 9/20, 9/28, 9/48, 47/02, 47/04, 47/10, 47/12, 47/38, C07D451/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
BIOSIS(STN), CAplus(STN), EMBASE(STN), MEDLINE(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 11-60482 A (Eisai Co., Ltd.), 02 March, 1999 (02.03.99), Full text (Family: none) | 1-5,8-20 |
| A | JP 1-203365 A (John Wyeth & Brother Ltd.), 16 August, 1989 (16.08.89), Full text & US 4983600 A & US 5106843 A & GB 2213816 A & GB 2225574 A & EP 323077 A1 & EP 361629 A2 | 1-20 |

[X] Further documents are listed in the continuation of Box C.   [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 June, 2005 (13.06.05) | 28 June, 2005 (28.06.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

INTERNATIONAL SEARCH REPORT

International application No.
PCT/JP2005/007564

C (Continuation). DOCUMENTS CONSIDERED TO BE RELEVANT

| Category* | Citation of document, with indication, where appropriate, of the relevant passages | Relevant to claim No. |
|---|---|---|
| A | JP 5-310749 A (Nisshin Flour Milling Co., Ltd.), 22 November, 1993 (22.11.93), Full text & US 5256656 A & US 5187166 A & EP 469449 A1 & CA 2047848 A | 1-20 |
| A | JP 2002-138051 A (Eisai Co., Ltd.), 14 May, 2002 (14.05.02), Full text (Family: none) | 1-20 |
| A | JP 2003-137778 A (Taiyo Pharmaceutical Industry Co., Ltd.), 14 May, 2003 (14.05.03), Full text (Family: none) | 1-20 |
| A | WO 2002/085901 A1 (PHARMACIA & UPJOHN CO.), 31 October, 2002 (31.10.02), Full text & JP 2004-526788 A & US 2003/055043 A1 & EP 1389208 A1 & CA 2439960 A | 1-20 |
| A | JP 2003-327533 A (Takeda Chemical Industries, Ltd.), 19 November, 2003 (19.11.03), Claims; Par. No. [0014] & EP 1420763 A1 & WO 2003/017980 A1 & CA 2448760 A | 3-14 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2721631 B